# EUROPEAN PATENT APPLICATION

(11) **EP 3 272 340 A1**
(43) Date of publication of application: **24.01.2018**
(21) Application number: 16191168.0
(22) Date of filing: 28.09.2016
(51) Int. Cl.: A61K 9/48

(54) **ACID RESISTANT CAPSULES**

(30) Priority: 22.07.2016 US 201662365661 P
(71) Applicant: Capsugel Belgium NV, 2880 Bornem (BE)
(72) Inventor: VANQUICKENBORNE, Stefaan Jaak, B-2820 Rijmenam (BE); HE, Xiongwei, 68280 Andolsheim (FR); FRIESEN, Dwayne, Bend, OR Oregon 97702 (US); Cape, Jonathan, Bend, OR Oregon 97702 (US)
(74) Representative: De Clercq & Partners

(57) **Abstract**

An acid resistant capsule comprises at least one hard capsule shell, said shell comprising: an enteric polymer having acid groups; a film-forming aid; and an alkaline material, wherein said alkaline material is present in an amount such that said carboxylic acid groups of said enteric polymer have a degree of ionization of less than 15%.

## Description

### FIELD

The present disclosure relates to new acid resistant hard capsules suitable for pharmaceutical use, a process for their manufacture and use of such capsules particularly but not exclusively for oral administration of pharmaceuticals, veterinary products, food and dietary supplements, to subjects selected from humans or animals.

### BACKGROUND

Hard pharmaceutical capsules are generally manufactured by using a dip molding process. In this process, pin molds are dipped into an aqueous-based film-forming composition. A film is formed by subsequently gelling the composition adhered on the pins. The film is then dried, stripped off the pins and cut to a desired length. Thus, capsule caps and bodies are obtained that can later be filled with a substance and telescopically joined together such that a filled, hard pharmaceutical capsule is obtained. For patent literature disclosing this process one can see e.g. US5264223, US5756123 and US5756123.

Pharmaceutical capsules are widely used in the pharmaceutical field as oral dosage forms for administration to humans and animals. For some applications, it is desirable that the capsules be acid resistant in order to remain intact in the stomach of patients and not to release the encapsulated content therein. Acid resistant capsules are thus useful for the administration of substances that are unstable in an acidic environment, or substances, like NSAIDs, that are associated with serious gastric side-effects.

Conventionally, the problem of imparting acid resistance to a capsule has been tackled by coating a non-acid resistant capsule with an enteric film. The enteric film comprises well-known acid resistant materials that have a pH-dependent water solubility. Typically, these materials are carboxylic group-containing polymers, such as cellulose acetate phthalate (CAP), hydroxypropyl methylcellulose phthalate (HPMCP), hydroxypropyl methylcellulose acetate succinate (HPMC-AS), carboxyl-containing acrylic copolymers and shellac. These materials are water insoluble under gastric conditions (conventionally simulated by pH 1.2) and readily water soluble under intestinal conditions (conventionally simulated by a pH of 6.8).

Drawbacks of the coating solution are typically represented by the complexity and costs of the manufacturing coating process, the high level of expertise needed to effectively perform it, the necessity to perform the coating at the end of the manufacturing cycle, i.e. once the capsules are already filled and, finally, the need for contacting the capsules with solvent-based coating compositions that may leave toxic solvent residues on the capsule surface after drying.

Attempts are also known to develop non-coated enteric hard pharmaceutical capsules, i.e. hard capsules whose shells already display gastric resistance and that, as such, do not need any coating step. One such method is a double dipping method, wherein conventional pins are dipped twice, at least one time in a solution of enteric polymer(s) dissolved in one or more organic solvents. The polymers used in double dipping methods are the same polymers used in conventional coating processes.

However, the double dipping process needs specifically developed production equipment which is extremely expensive. Additionally, the problem of using organic solvents in the dipping solution is not overcome which still causes serious concerns about environment and health security.

Other attempts to provide bulk acid resistant capsules have been made by combination of Hydroxypropyl-Methyl-cellulose (HPMC) and gellan gum at specific ratios, as exemplified in US2015050334A1.

Further alternative attempts to provide acid resistant capsules have been made by combination of enteric polymers such as HPMCAS or HPMCP with capsule forming aids such as HPMC or Methyl Cellulose (MC) in combination with a neutralizing agent, as exemplified in US8710105B2.

However, it has been found that addition of a neutralizing agent, although being beneficial in the capsule making process by enabling effective solubilization of the enteric polymer, may adversely impact the dissolution profile of the capsule particularly when administering with water (typical in oral administration thereof). Without wishing to be bound by theory, it is believed that once the capsule shells are contacted with water the enteric polymer effectively re-solubilizes upon hydration of the residual alkaline material (just like when in the aqueous state during capsule manufacture) and consequently may alter the release profile of the medicaments contained in the capsule.

A need therefore remains to provide acid resistant capsules wherein the acid resistance and/or enteric properties thereof are improved and/or retained (i.e. not adversely affected) even at higher pH such as when administering the capsule with water.

### SUMMARY

An acid resistant capsule comprises at least one hard capsule shell, the shell comprising: at least one enteric polymer having carboxylic acid groups; a film-forming aid; and an alkaline material, wherein the alkaline material is present in an amount such that the carboxylic acid groups of the enteric polymer have a degree of ionization of less than 15%.

In one embodiment, the degree of ionization is less than 12%, preferably less than 10%, more preferably from 0.1 % to 9%.

In one embodiment, the alkaline material is selected from the group consisting of ammonia (also referred to as ammonium hydroxide), ethanolamine, diethanolamine, triethanolamine, lithium hydroxide, sodium hydroxide, potassium hydroxide, calcium hydroxide, magnesium hydroxide, sodium phosphate, sodium carbonate, sodium citrate, sodium ascorbate, lysine, arginine, cationic polymers, and mixtures thereof.

In one embodiment, the alkaline material is volatile.

In one embodiment, the alkaline material is selected from the group consisting of ammonia and ethanolamine. In a preferred embodiment the alkaline material is ammonia.

In one embodiment, the enteric polymer is selected from the group consisting of hydroxypropyl methylcellulose acetate succinate (HPMCAS), hydroxypropyl methylcellulose phthalate (HPMCP), cellulose acetate phthalate (CAP), acrylic polymers, polyvinyl acetate phthalate (PVAP), and mixtures thereof.

In one embodiment, the film-forming aid is selected from the group consisting of hydroxypropyl methylcellulose (HPMC), methylcellulose (MC), gellan gum, carrageenan, and mixtures thereof, preferably HPMC, MC, and mixtures thereof, more HPMC.

In one embodiment, the weight ratio of the enteric polymer to the film-forming aid is from 1.5 to 3.5, preferably from 1.8 to 2.8, more preferably from 2 to 2.5.

In one embodiment, the alkaline material is present in the capsule in an amount of less than 7000 ppm, preferably less than 6500 ppm, more preferably from 1000 ppm to 6400 ppm, at room temperature.

In one embodiment, an acid resistant capsule consists essentially of: water present in an amount from 1wt% to 20wt%; HPMCAS present in an amount from 40 wt% to 90wt%; a film-forming aid selected from the group consisting of HPMC and MC present in an amount of from 10wt% to 50wt%; and an alkaline material selected from the group consisting of ammonia and ethanolamine present in an amount of less than 1wt%, more preferably less than 0.5wt%, wherein said alkaline material is present in an amount such that the carboxylic acid groups of said HPMCAS have a degree of ionization of less than 15%.

A dip-molding process for the manufacture of acid resistant hard capsule shells comprises the steps of:
a) providing an aqueous composition comprising an enteric polymer having carboxylic acid groups, a film-forming aid, and an alkaline material, the alkaline material being present in a sufficient amount so that the enteric polymer is homogeneously dispersed in the aqueous composition;
b) dipping mold pins in the aqueous composition;
c) extracting the mold pins from the aqueous composition such that a film forms over each of the mold pins;
d) drying the film to form a solid coating on each of the mold pins;
e) removing the solid coating from the mold pins to provide capsule shells; and
f) reducing the amount of the alkaline material in the capsule shells such that the carboxylic acid groups of the enteric polymer in the capsule shells have a degree of ionization of less than 15%.

In one embodiment, the step (f) of reducing the amount of alkaline material is an active removal step.

In one embodiment, the alkaline removal step (f) comprises the step of gently heating the capsule shells at a temperature of from 30°C to 80°C, preferably from 45°C to 75°C, preferably from 35°C to 70°C, and at a relative humidity (RH) of from 3% to 85%, preferably from 20% to 80%, more preferably from 30% to 70%. Alternatively, the RH may be from 3% to 15%, preferably from 4% to 10%, however at such lower RH's it may be desirable to introduce a further moisture re-equilibration step to adjust the LOD (Loss on Drying) of the capsule shells to the desired level (typically of about 3-5% LOD).

In one embodiment, the alkaline removal step (f) further comprises tumbling the capsule shells, preferably simultaneously to the gentle heating.

In one embodiment, the alkaline material removal step (f) is carried out for a predetermined period of time ranging from 1 hour to 20 hours, preferably from 1.5 hours to 18 hours, more preferably from 2 hours to 16 hours.

In one embodiment, the acid resistant hard capsule are used to delay the release of one or more medicaments contained in the capsule when contacted with unbuffered water, preferably such that less than 20% of the medicament is released after 60 minutes in demineralized water.

In one embodiment, the acid resistant hard capsule is used for enteric release of one or more medicaments contained in the capsule, typically such that less than 10% of the medicament is released after 2 hours in a simulated gastric buffer at a pH of 1.2 and that at least 80% of the medicament is released after 45 minutes in a simulated intestinal buffer at a pH of 6.8, as measured using a USP Dissolution Apparatus 2 (paddle) method.

### BRIEF DESCRIPTION OF THE DRAWINGS

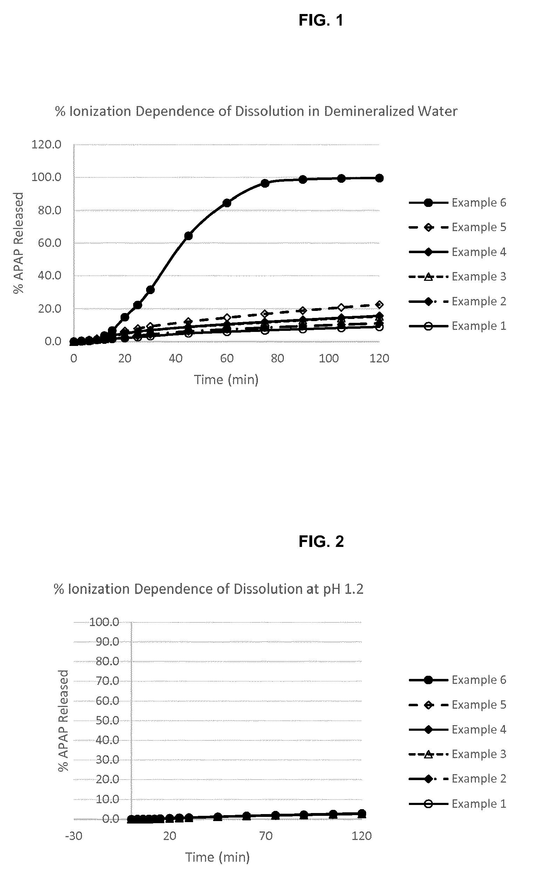

Fig. 1 shows a series of in vitro dissolution curves of release of acetaminophen from the acid resistant capsules of the examples in demineralized water.
Fig. 2 shows a series of in vitro dissolution curves of release of acetaminophen from the acid resistant capsules in gastric media.

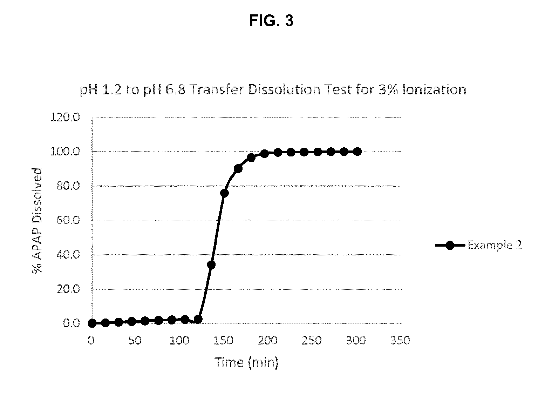

Fig. 3 shows a series of in vitro dissolution curves of release of acetaminophen from the acid resistant capsules for one of the examples in a gastric to intestinal transfer test.

### DETAILED DESCRIPTION

By the term "a" and/or "an" when describing a particular element, it is intended "at least one" of that particular element.

By the term "medicament", it is intended a drug, active pharmaceutical ingredient, or the like comprising one or more compounds providing one or more therapeutic or prophylactic benefits to a subject; the terms "medicament" and "drug" may be used interchangeably herein.

By the term "hard shell" or "hard capsule shell", it is intended a shell that is capable of maintaining a shape so as to be filled with and encapsulate a medicament using conventional capsule filling equipment.

By the term "two-piece" hard capsules as used herein, it is intended a hard capsule made of two shells, typically a cap shell telescopically engageable over a body shell.

By the term "degree of ionization" as used herein, is meant the percentage of the acid groups of the enteric polymer (e.g. the succinic acid groups present in the HPMCAS polymer) that are negatively charged (e.g., the percentage that have been neutralized with an alkaline material (such as a base) or are not in a protonated state).

By the term "volatile" as used herein, means materials capable of readily evaporating at room temperature and pressure.

By the term "wt%" as used herein, is meant the weight of a material relative to the total weight of the composition or capsule as the case may be.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm" (i.e. every value in a practical range close to 40 mm).

Various embodiments will now be described to provide an overall understanding of the principles of the structure, function, manufacture, and use of dosage form articles and methods disclosed herein. One or more examples of these embodiments are illustrated in the accompanying figures. Those of ordinary skill in the art will immediately understand that features described or illustrated in connection with one example embodiment can be combined with the features of other example embodiments without generalization from the present disclosure.

### DOSAGE FORM ARTICLES

The acid-resistant capsules are solid dosage forms in which the medicament is encapsulated in a hard container or shell. Normally, the capsule shells comprise caps and bodies having a side wall, an open end and a closed end. The length of the side wall of each of the shell parts is generally greater than the capsule diameter. Thus, the hard capsules of the present disclosure do not structurally depart from the conventional definition of hard capsules. "Capsule" refers to both empty and filled capsules.

The acid resistant capsule contains at least three materials: an enteric polymer having acid groups; a film-forming aid; and an alkaline material. The acid resistant capsules have improved acid resistance due to the removal or neutralization of the alkaline material in the capsule after formation of the capsule shell. While the alkaline material is present in the aqueous dipping composition in a sufficient amount to solubilize the enteric polymer, the alkaline material is present in the final capsule in an amount such that the acid groups of the enteric polymer have a degree of ionization of less than 15%.

Enteric polymers suitable for use in the acid resistant capsule are any which are amenable to manufacture using a conventional dip-molding process for making capsules. In one embodiment, the enteric polymer is selected from the group consisting of hydroxypropyl methylcellulose acetate succinate (HPMCAS), hydroxypropyl methylcellulose phthalate (HPMCP), cellulose acetate phthalate (CAP), acrylic polymers, polyvinyl acetate phthalate (PVAP), and mixtures thereof. Preferred enteric polymers for use herein are selected from the group consisting of HPMCAS, HPMCP, and CAP. In one embodiment, the enteric polymer is HPMCAS. In one embodiment the acid groups on the enteric polymer are carboxylic acid groups. The term "enteric polymer" as used herein and in the claims includes both a single species of polymer and mixtures of one or more enteric polymers.

A method for determining the pKₐ of an enteric polymer includes dispersing an amount of the polymer sufficient to provide a concentration of about 0.01 molar acidic groups in 100 milliliters of a 50% demineralized water/50% methanol/ 0.1 molar potassium chloride solution at 23°C. Stirring is maintained throughout the dispersion and dissolution process. A pH electrode, calibrated at room temperature with pH 4.0, 7.0 and 10.0 buffer standards is then immersed into the solution and stirring is maintained. A strong acid, such as hydrochloric acid, is then added to the polymer solution in a quantity sufficient to bring the pH to about 3.0. The pH of the polymer solution is then recorded as a function of the quantity of an added base titrant, such as sodium hydroxide, which is typically added in about 10 micromole aliquots until the pH value of about 9.0 is achieved. A plot of pH versus moles of added base consists of an acidic region where the pH rises sharply as a function of added base, a buffering region where the pH remains relatively flat as a function of added base, and an alkaline region where the pH again rises sharply as a function of added base. The pKₐ is taken as the pH at the midpoint between the inflections in the aforementioned curve that define the buffering region.

The enteric polymer is present in a sufficient amount so as to provide the desired level of acid resistance. Typically the enteric polymer is present in the finished capsule in an amount of from 40 to 90 wt% and preferably 50 to 80 wt%.

The acid groups on the enteric polymer in the finished acid resistant capsule have a degree of ionization that is less than 15%. By the term "degree of ionization" as used herein, is meant the percentage of the acid groups of the enteric polymer (e.g. the succinic acid groups present in the HPMCAS polymer) that are in the ionized state (e.g., neutralized with an alkaline material (such as a base), or not in a protonated state). This parameter is calculated as degree of ionization = 100/(1 + 10^{pKa-pH}), where the pKₐ refers to the negative logarithm of the acid ionization constant for the carboxylic acid groups on an enteric polymer, and pH refers to the negative logarithm of the proton or hydronium concentration in solution as measured using a calibrated pH electrode. In cases where one or more enteric polymers are present, the degree of ionization value is taken as the weighted sum of the individual degree of ionization values from each polymer, where the weighting of each value is given by the fractional amount of each polymer with respect to the to the total amount of enteric polymer present in the composition.

To determine the degree of ionization of the enteric polymer in the finished capsule, a pH is determined by dissolving finished capsule(s) to a concentration of 10 mg/mL in a stirring solution of 1:1 MeOH:H2O at 23°C and then measuring the pH of the resulting solution with a pH electrode previously calibrated with pH 4.0, 7.0, and 10.0 standard buffers. It is assumed that the ratio of ionized to non-ionized acidic groups on the polymer does not change upon dissolution into a non-buffered solvent. This pH value is used in the equation above. Similarly, to determine the degree of ionization of the enteric polymer in the aqueous solution, the pH of the aqueous solution is measured, and that value is used in the equation above.

For example, in the case of the acid groups of polyvinyl acetate phthalate, the pKa is about 5.0. Thus, the degree of ionization as a function of pH is 1% at pH 3.0; 9% at pH 4.0; 50% at pH 5.0; and 91% at pH 6.0. In general, the degree of neutralization of the enteric polymer in the aqueous composition is from 1 to 90wt%, preferably from 30 to 90wt%, even more preferably 60 to 90wt%.

The acid resistant capsule also contains a film-forming aid. The film-forming aid is any material amenable for use in a conventional dip-molding process that improves the setting of the enteric polymer on the dip-molding pin to enable film formation. The term "film-forming aid" includes single species of materials or mixtures of materials. In one embodiment, the film-forming aid is selected from the group consisting of hydroxypropyl methylcellulose (HPMC), methylcellulose (MC), gellan gum, carrageenan, and mixtures thereof. In one embodiment, the film-forming aid is selected from the group consisting of HPMC, MC, and mixtures thereof. The film-forming aid is most preferably HPMC.

The amount of film-forming aid present in the finished capsule depends on the setting properties of the film-forming aid. When the film-forming aid is HPMC or MC, the film-forming aid is present in the final capsule in an amount ranging from 10 to 50 wt% and preferably 20 to 40 wt%. In general, when the film-forming aid is HPMC or MC, the weight ratio of the enteric polymer to the film-forming aid is from 1.5 to 3.5, preferably from 1.8 to 2.8, and more preferably from 2 to 2.5. One advantage to using HPMC or MC is the fact that additional gelling agents like carrageenan, gums (e.g. gellan gum) and the like, can be omitted from the formulation.I In a preferred embodiment, the finished capsules described herein are free of gelling agents and consist essentially of water, the enteric polymer(s), a film-forming aid selected from the group consisting of HPMC and MC, and one or more alkaline materials.

When the film-forming aid has stronger setting properties, such as gellan gum or carrageenan, less film-forming aid is needed. For example, when the film-forming aid is gellan gum or carrageenan, the film-forming aid is present in the final capsule in an amount ranging from 0.1 to 5.0 wt% and preferably 0.5 to 2.0 wt%

The finished capsule also contains an alkaline material. However, the purpose of the alkaline material is as a processing aid during manufacture of the capsule, and has no function in the finished capsule. In one embodiment, the alkaline material is selected from the group consisting of ammonia, ethanolamine, diethanolamine, triethanolamine, lithium hydroxide, sodium hydroxide, potassium hydroxide, calcium hydroxide, magnesium hydroxide, sodium phosphate, sodium carbonate, sodium citrate, sodium ascorbate, lysine, arginine, cationic polymers, and mixtures thereof.

In a preferred embodiment, the alkaline material is volatile. In a highly preferred embodiment, the alkaline material is a volatile material, preferably having a vapor pressure of greater than 0.04 kPa, preferably greater than 4 kPa, more preferably greater than 5 kPa, even more preferably greater than 10 kPa, at room temperature (i.e. 25°C). Volatile alkaline materials are preferred because they can be removed through relatively benign methods from the capsule shell. In one embodiment in which the alkaline material is volatile, the alkaline material is selected from the group consisting of ammonia, and ethanolamine. A preferred alkaline material is ammonia.

As discussed above, the function of the alkaline material is as a processing aid during manufacture to solubilize the enteric polymer in the aqueous dipping composition, but otherwise has no function in the finished capsule. Accordingly, in one embodiment, the alkaline material is present in the finished capsule in an amount of less than 7000 ppm, preferably less than 6500 ppm, and more preferably less than from 1000 ppm to 6400 ppm, at room temperature. In one embodiment, the amount of alkaline material present in the finished capsule is less than 7wt%, may be less than 2wt%, and may be less than 1 wt%, and may be less than 0.75wt%. In a preferred embodiment, the amount of alkaline material present in the finished capsule is less than 0.5wt%.

The hard capsule shells may optionally further include other minor components conventionally used in capsules or that are used in the aqueous composition for dipping and that remain as part of the finished capsule. Examples of such materials include surfactants, de-foaming aids, anti-oxidants, viscosity modifiers, gelling agents, gelling aids, lubricants and plasticizers.

The hard capsule shells also contain residual water. Typically such shells comprise, for example, less than 25wt%, preferably less than 20wt%, more preferably from 0wt% to 14wt%, even more preferably from 1wt% to 10wt%, and more preferably from 2wt% to 7wt% water by weight.

In one embodiment the capsule comprises a colorant or opacifier such as titanium dioxide and/or colorants such as mineral colorants, natural colorants, and tar colorants, and the like. In one embodiment, the coloring agent may be selected from azo-, quinophthalone-, triphenylmethane-, xanthene- or indigoid dyes; iron oxides or hydroxides; titanium dioxide; or natural dyes and mixtures thereof. Further examples are patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, anthocyanines, turmeric, cochineal extract, chlorophyllin, canthaxanthin, caramel, betanin and Candurin® pearlescent pigments. Candurin® is manufactured and marketed by Merck KGaA, Darmstadt, Germany and consist of titanium dioxide and/or iron oxide - approved food and pharmaceutical colorants in many countries - and potassium aluminium silicate as color carrier. The latter is a natural, also widely approved, silicate also known under the name of 'mica'.

In one embodiment, the acid-resistant capsules are used to delay the release of one or more medicaments contained in said capsule when contacted with unbuffered water, preferably such that less than 20wt% of said medicament is released after 60 minutes in demineralized water. The dissolution performance of the capsules is determined using a USP II dissolution apparatus. A capsule is filled with a medicament, mounted in a sinker and placed into the USP II dissolution apparatus filled with one liter of demineralized water held at a temperature of 37°C. The paddle speed is set at 50 rpm. Replicate measurements are performed to provide average release of medicament at selected time points.

In one embodiment, the acid-resistant capsules provide improved resistance to gastric dissolution media, whether in vivo or in vitro, relative to gelatin capsules and capsules formed from non-enteric polymers such as HPMC. In one embodiment, the capsule shells comprise a dissolution release of less than about 10wt% of the total encapsulated medicament after a time of about 2 hours when exposed to a simulated gastric media of about pH 1.2 held at a temperature of 37°C in which the capsule is mounted in a sinker and placed into a USP II dissolution apparatus with the paddle speed set at 50 rpm.

However, the acid-resistant capsules dissolve or disintegrate when exposed to intestinal buffer media, whether in vivo or in vitro, so as to rapidly release the encapsulated medicament. In one embodiment, the dissolution release is about 80wt% of the total encapsulated medicament a time of about 45 minutes after administration to simulated intestinal buffer media of about pH 6.8 held at a temperature of 37°C in which the capsule is mounted in a sinker and placed into a USP II dissolution apparatus with the paddle speed set at 50 rpm.

In one embodiment, capsule shells have bulk enteric properties when they have dissolution profiles that match both the dissolution profile in simulated gastric dissolution media and simulated intestinal dissolution media as reported above. A dissolution test for evaluating enteric release may be performed as follows. After filling a medicament into a finished capsule, the capsule is place in a pH 1.2 buffer (0.063 M HCl) held at a temperature of 37°C for two hours in a USP II dissolution apparatus. At two hours, the pH of the dissolution bath is brought to pH6.8 with the addition of a disodium phosphate solution to bring the final phosphate concentration to 0.2 molar. Concentration of the release medicament is measured at various time points to determine the amount of medicament released in gastric and intestinal dissolution media.

### METHODS OF MAKING

Capsules according to the present disclosure are typically made via a dip-molding process.

Dip-molding processes for making acid-resistant two-piece hard capsules comprise the steps of:
a) providing an aqueous composition comprising an enteric polymer, a flim-forming aid, and an alkaline material, the alkaline material being present in a sufficient amount so that the enteric polymer is homogeneously dispersed in the aqueous composition;
b) dipping mold pins in the composition;
c) extracting the mold pins from the dipping composition such that a film is formed over each of the mold pins;
d) drying the film to form a solid coating on the mold pins;
e) removing the solid coating from the mold pins to provide capsule shells; and
f) reducing the amount of the alkaline material in the capsule shells.

Steps (a) to (f) are typically to be performed in the order they are presented. However, step (f) may be conducted prior to step (e) wherein the capsule shells are still on the pins or may be conducted both while the capsule shells are on the pins and also after the capsule shells are removed from the pins.

As described herein, "finished capsule shells" are those capsule shells for which step (f) has been completed.

In step (a) the aqueous composition is prepared by combining water, the enteric polymer, the film-forming aid, and the alkaline material. In one embodiment, the aqueous composition comprises 10%wt to 20%wt of enteric polymer such as HPMCAS, and 3%wt to 10%wt of the film-forming aid HPMC. Step (a) may comprise an optional adjustment of the film-forming aid (or enteric polymer) concentration that can be performed to meet the desired viscosity. Preferred viscosities of the aqueous composition are in the range of about 1000 to 3000 mPa·s (cps), typically of about 1500 to 2500 mPa·s (cps), at room temperature and measured with a Brookfield Model DV-II viscometer.

The alkaline material is present in a sufficient amount to solubilize the enteric polymer in the aqueous composition so that the enteric polymer is homogeneously dispersed in the aqueous composition, thereby providing a homogenous film on the mold pins. Typically the alkaline material is added in a sufficient amount to result in a degree of ionization of the carboxylic acid groups on the enteric polymer(s) in the aqueous composition that ranges from 1 to 90wt%, preferably from 30 to 90wt%, even more preferably 60 to 90wt%. The amount of alkaline material present preferably results in an aqueous composition of the enteric polymer with no visible solids observable by naked eye after stirring. In a preferred embodiment, the aqueous composition has a pH of from 5.7 to 8, more preferably from 5.9 to 6.5.

In addition, the aqueous compositions (and resulting hard capsule shells) may optionally further include other minor components such as titanium dioxide and/or colorants such as mineral colorants, natural colorants, and tar colorants, anti-oxidants and the like. In one embodiment, the coloring agents, or mixtures thereof are present in an amount ranging from about 0 to about 5% by weight, e.g., from about 0 to about 2.5% by weight, and from about 0 to about 1.5% by weight over the total weight of the aqueous composition.

In step (b) mold pins are dipped in the aqueous composition. The relative temperatures of the aqueous composition and mold pins are chosen to result in film formation on the mold pins once the mold pins are dipped into the aqueous composition. In a preferred embodiment, the film-forming aid undergoes thermogelation and is MC or HPMC. In this embodiment the aqueous composition at the time the mold pins are dipped is kept at a temperature of from 25°C to 45°C, preferably from 30°C to 40°C. In step (c), the dipping composition is maintained at a temperature of 10°C to 1.0°C, preferably 6°C to 2.0°C, below its gelling temperature, typically the gelling temperature being from 30°C to 60°C. For example, if a dipping composition has a gelling temperature of about 36.0°C, it can be maintained at a temperature of for example about 34.0°C. Typically, in such embodiments film forming is achieved by thermogelation and the mold pins are pre-heated to a temperature that is greater than the gelation temperature of the aqueous composition prior to the dipping step. The temperature range of the pre-heated pins is 55-100°C, meaning that this is the mold pin temperature when mold pins are dipped. Preferably the temperature of the mold pins is 60-95°C, more preferably 70-95°C, more preferably from 80°C to 95°C, more preferably 85-95°C, even more preferably 90-95°C. It is preferred that such temperature be chosen according to the desired capsule size. By "according to the capsule size" it is meant that the smaller the pin dimension, the higher the temperature.

The use of HPMC or MC therefore has the advantage of enabling a relatively lower composition temperature of the aqueous composition during the dipping process, wherein the pins are heated to a temperature warmer than the aqueous composition. In addition, such film-forming polymers have the advantage of reducing the overall amount of alkaline material needed to fully neutralize the enteric polymer in the aqueous composition. Thus the thermogelation process with HPMC, where the dip composition is kept at a temperature below gelation and rather the mold pins are heated at temperatures above gelation prior immersion therein, is particularly preferred and selected for generating physically stable hard capsules described herein.

Alternatively, the film-forming aid may be a material such as carageenan, or gellan gum, which increases in viscosity or even gels upon cooling. When such film-forming aids are used, a "cold gelation" process is used in which the aqueous composition is maintained at a warm temperature and the mold pins are maintained at a lower temperature than the aqueous composition when dipped into the aqueous composition.

In step (c), the mold pins are extracted from the aqueous composition such that a film is formed over each of the mold pins. After being withdrawn from the aqueous composition, the mold pins can be turned from a "top- down" dipping position to a "top-up" drying position according to conventional capsule dip molding processes. In this step the pins are rotated about a horizontal axis of about 180° with respect to the dipping position of step (c).

In step (d), the film on the mold pins is dried. The purpose of the drying step (d) is to reduce the water content (also refered to herein as "moisture") in the capsule shells on the mold pins. In one embodiment, drying occurs at a temperature above the gelling temperature of the aqueous composition so as to obtain molded capsule shells on the pins. The drying step is preferably carried out at a temperature of less than 65°C, preferably less than 60°C, even more preferably from 40°C to 55°C. Step (d) is typically carried out for a period of time of from 30 to 60 minutes, preferably not exceeding the above identified temperatures. Generally, the water content in the molded capsule shells is reduced from around 80% to around 3-7% by weight, based on the total weight of the molded capsule shells (measured at room conditions).

However, step (d) may equally comprise a multi-step heating wherein the shells are subjected to a first drying temperature for a first drying time followed by a second drying temperature for a second drying time, wherein the first drying temperature is from 60°C to 80°C, the first drying time is from 1 minute to 15 minutes, the second drying temperature is from 20°C to 50°C, and the second drying time is from 29 to 60 minutes.

In step (e) the solid coating is removed from the mold pins to provide capsule shells. The solid coating may be removed using any conventional manufacturing technique. The mold pins may be lubricated in order to facilitate removal of the capsule shells.

In step (f) the amount of the alkaline material is reduced in the capsule shells. This reduces the degree of ionization of the acid groups on the enteric polymer in the finished capsule relative to the degree of ionization of the acid groups on the enteric polymer in the aqueous composition. This improves the dissolution properties of the finished capsule, such as by inhibiting release in unbuffered water but releasing in intestinal fluid.

The amount of alkaline material may be reduced by either removing the alkaline material from the capsule, or neutralizing the alkaline material in the capsule shell. The step of reducing the amount of alkaline material may occur while the films are over the mold pins or after the shells have been formed and already stripped from the mold pins or a combination of both.

In an embodiment, the step (f) of reducing the amount of the alkaline material is an active removal step (i.e. not a passive removal step). For example, the removal step may comprise gentle heating and/or tumbling of the shells for a predetermined period of time. In one embodiment, the alkaline removal step comprises the step of gently heating the capsule shells at a temperature of from 30°C to 80°C, preferably from 45°C to 75°C, and typically at a relative humidity (RH) of from 3% to 85%, preferably from 20% to 80%, more preferably from 30% to 70% for a predetermined period of time (preferably from 4 to 24 hours).

In another embodiment, the step (f) of reducing the amount of the alkaline material may comprise the step of laying the capsules on a surface (e.g. a tray or moving belt) exposed to a fluid (e.g. air) typically at the temperatures and relative humidity parameters described above. Preferably, a vibration or tapping is applied to the surface such to shake the capsules carried thereon. An advantage is that the alkaline removal time is thus reduced.

In another embodiment, the step (f) of reducing the amount of the alkaline material comprises the step of exposing the capsule shells to a vacuum (e.g. in a vacuum drier).

In an embodiment, the step (f) of reducing the amount of the alkaline material comprises the step of neutralizing the alkaline material in the capsule by exposure to an acidic material. In one embodiment, the alkaline material in the capsule is neutralized by contacting the capsule shells with a flow of an acidic gas (e.g. CO₂ or acetic acid). Such may advantageously further normalize the pH of the capsule shells.

In an embodiment, the step (f) of reducing the amount of the alkaline material in the capsule shell comprises the step of contacting the capsule shells with acidic water, typically at a pH of from 0 to 3. The contacting method may comprise, for example, spraying the capsules with an acidic solution or dipping the capsules into an acidic solution. Such contacting may occur while the capsule shells are on the pins or after removal from the pins.

A person skilled in the art will understand that a combination of any of the above described embodiments of the step of reducing the alkaline material may be made to optimize the degree of ionization of the enteric polymer of the shells. Reference to reducing the amount of alkaline material in the capsule is not limited to the actual removal of the alkaline material but further also extends to the neutralization thereof.

The step of reducing the amount of alkaline material in the capsule is conducted such that the degree of ionization of the carboxylic acid groups of the enteric polymer(s) are at or below the desired level. In general the alkaline removal step achieves a degree of ionization of the enteric polymer that is less than 15%, preferably less than 12%, more preferably less than 10%, and even more preferably less than 9%.

Thus in one aspect, the present disclosure relates to a process for the manufacture of acid-resistant capsules comprising hydroxypropyl methyl cellulose acetate succinate (HPMCAS) according to a dip coating process, characterized in that it comprises the steps of: (a) providing an aqueous composition comprising 10%wt to 20%wt of HPMCAS, 3%wt to 10%wt of HPMC, and an alkaline material in an effective amount to neutralize at least 10% w/w of the acid groups of the HPMCAS in the composition; (b) pre-heating dipping pins so that they are at 55-100°C when dipped into the aqueous composition and dipping the pre-heated dipping pins into the aqueous composition maintained at a temperature of 1°C to 10°C below its gelling temperature; (c) withdrawing the dipping pins from the aqueous composition obtaining a film on the dipping pins; (d) drying the film on the dipping pins at a temperature above the gelling temperature of the aqueous composition so as to obtain molded capsule shells on the pins; (e) removing the molded capsule shells from the pins to provide capsule shells; and and (f) reducing at least 50% by weight of the alkaline material initially present in the aqueous composition of the alkaline material from the hard capsule shells. Unless otherwise indicated, with wt% values being by weight of the aqueous composition.

The molded capsule shells mentioned to above, generally refer to both bodies and caps, depending on the shape of the mold pin. Thus, after step (e) or (f) the dried capsule shells on the dipping pins can be processed according to conventional steps. This means that in general after step (e) or (f), the capsule shells (bodies and caps) are stripped from the pins. This step can be followed by cutting the stripped shells to a desired length.

Typically, hard capsule dip-molding manufacturing processes encompass an additional step of lubricating the pins so as to make it easier to strip the capsule shells from the pins.

Lubrication is normally achieved via the application of a demolding agent to the pins surface.

### METHODS OF USE

Drugs (i.e. medicaments) suitable for use in the dosage form articles described herein may take any form and be for any treatment of a human or animal subject. This includes not only pharmaceutical compounds but also dietary supplements such as vitamins, minerals and the like. The drug may be in a state selected from solid or liquid, preferably solid, at room temperature and atmospheric pressure, and comprises one or more active compounds. The medicament may be solid and in the form of spray dried dispersions, pellets, granules and the like.

Suitable compounds (and generally encompassed by the term "medicament" as used herein) for delivery according to the disclosure include, but are not limited to, particulate, powder, waxy, liquid, and/or pellet forms of the following:
a) pharmaceuticals (also called pharmaceutical actives) such as betamethasone, thioctic acid, sotalol, salbutamol, norfenefrine, silymahn, dihydroergotamine, buflomedil, etofibrate, indomethacin, oxazepam, acetyldigitoxins, piroxicam, halopehdol, isosorbide mononitrate, amithptyline, diclofenac, nifedipine, verapamil, pyritinol, nitrendipine, doxy- cycline, bromhexine, methylprednisolone, clonidine, fenofibrate, allopurinol, pirenzepine, levothyroxine, tamoxifen, metildigoxin, o-(B-hydroxyethyl)-rutoside, propicillin, aciclovir-mononitrate, paracetamolol, naftidrofuryl, pentoxifylline, propafenone, acebutolol, 1- thyroxin, tramadol, bromocriptine, loperamide, ketofinen, fenoterol, ca-dobesilate, propranolol, minocycline, nicergoline, ambroxol, metoprolol, B-sitosterin, enalaprilhydro- genmaleate, bezafibrate, isosorbide dinitrate, gallopamil, xantinolnicotinate, digitoxin, flunitrazepam, bencyclane, depanthenol, pindolol, lorazepam, diltiazem, piracetam, phenoxymethylpenicillin, furosemide, bromazepam, flunarizine, erythromycin, metoclo- pramide, acemetacin, ranitidine, biperiden, metamizol, doxepin, dipotassiumchloraze- pat, tetrazepam, estramustinephosphate, terbutaline, captopril, maprotiline, prazosin, atenolol, glibenclamid, cefaclor, etilefrin, cimetidine, theophylline, hydromorphone, ibu- profen, primidone, clobazam, oxaceprol, medroxyprogesterone, flecainide, Mg- pyhdoxal-5-phosphateglutaminate, hymechromone, etofyllineclofibrate, vincamine, cin- narizine, diazepam, ketoprofen, flupentixol, molsidomine, glibornuhde, dimethindene, melperone, soquinolol, dihydrocodeine, clomethiazole, clemastine, glisoxepid, kallidino- genase, oxyfedhne, baclofen, carboxymethylcystsin, thioredoxin, betahistine, 1-tryptophan, myrtol, bromelain, prenylamine, salazosulfapyridine, astemizole, sulpiride, benzerazid, dibenzepin, acetylsalicylic acid, miconazole, nystatin, ketoconazole, sodium picosulfate, colestyramate, gemfibrozil, rifampin, fluocortolone, mexiletine, amoxicillin, terfenadine, mucopolysaccharidpolysulfuric acid, triazolam, mianserin, tiaprofensaure, ameziniummethylsulfate, mefloquine, probucol, quinidine, carbamazepine, Mg-1- aspartate, penbutolol, piretanide, amitriptyline, caproteron, sodium valproinate, me- beverine, bisacodyl, 5-amino-salicyclic acid, dihydralazine, magaldrate, phenprocou- mon, amantadine, naproxen, carteolol, famotidine, methyldopa, auranofine, estriol, nadolol, levomepromazine, doxorubicin, medofenoxat, azathioprine, flutamide, norfloxacin, fendiline, prajmaliumbitartrate, aescin acromycin, anipamil, benzocaine, [beta]- carotene, cloramphenicol, chlorodiazepoxid, chlormadinoneacetate, chlorothiazide, cinnarizine, clonazepam, codeine, dexamethasone, dicumarol, digoxin, drotaverine, gramicidine, griseofulvin, hexobarbital hydrochlorothiazide, hydrocortisone, hydroflumethiazide, ketoprofen, lonetil, medazepam, mefruside, methandrostenolone, sulfaperine, nalidixic acid, nitrazepam, nitrofurantoin, estradiol, papaverine, phenacetin, phenobarbi- tal, phenylbutazone, phenytoin, prednisone, reserpine, spironolactine, streptomycin, sulfamethizole, sulfamethazine, sulfamethoxoazole, sulfamethoxydiazinon, sulfathiazole, sulfisoxazole, testosterone, tolazamide, tolbutamide, trimethoprim, tyrothricin, antacids, reflux suppressants, antiflatulents, antidopaminergics, proton pump inhibitors, H2- receptor antagonists, cytoprotectants, prostaglandin analogues, laxatives, antispasmodics, antidiarrhoeals, bile acid sequestrants, opioids, beta-receptor blockers, calcium channel blockers, diuretics, cardiac glycosides, antiarrhythmics, nitrates, antianginals, vasoconstrictors, vasodilators, ACE inhibitors, angiotensin receptor blockers, alpha blockers, anticoagulants, heparin, antiplatelet drugs, fibrinolytic, anti-hemophilic factor, haemostatic drugs, hypolipidaemic agents, statins, hypnotics, anaesthetics, antipsychotics, antidepressants (including tricyclic antidepressants, monoamine oxidase inhibitors, lithium salts, selective serotonin reuptake inhibitors), anti-emetics, anticonvulsants, an- tiepileptics, anxiolytics, barbiturates, movement disorder drugs, stimulants (including amphetamines), benzodiazepine, cyclopyrrolone, dopamine antagonists, antihistamines, cholinergics, anticholinergics, emetics, cannabinoids, 5-HT antagonists, analgesics, muscle relaxants, antibiotics, sulfa drugs, aminoglycosides, fluoroquinolones, bronchodilators, NSAIDs, anti-allergy drugs, antitussives, mucolytics, decongestants, corticosteroids, beta-receptor antagonists, anticholinergics, steroids, androgens, antian- drogens, gonadotropin, corticosteroids, growth hormones, insulin, antidiabetic drugs (including sulfonylurea, biguanide/metformin, and thiazolidinedione), thyroid hormones, antithyroid drugs, calcitonin, diphosponate, vasopressin analogs, contraceptives, follicle stimulating hormone, luteinising hormone, gonadotropin release inhibitor, progestogen, dopamine agonists, oestrogen, prostaglandin, gonadorelin, clomiphene, tamoxifen, di- ethylsti I bestrol , antimalarials, anthelmintics, amoebicides, antivirals, antiprotozoals, vaccines, immunoglobulin, immunosuppressants, interferon, monoclonal antibodies, and mixtures thereof;
b) vitamins, e.g., fat-soluble vitamins such as vitamins A, D, E, and K, and water soluble vitamins such as vitamin C, biotin, folate, niacin, pantothenic acid, riboflavin, thiamin, vitamin B6, vitamin B12, and mixtures thereof;
c) minerals, such as calcium, chromium, copper, fluoride, iodine, iron, magnesium, manganese, molybdenum, phosphorus, potassium, selenium, sodium (including sodium chloride), zinc, and mixtures thereof;
d) dietary supplements such as herbs or other botanicals, amino acids, and substances such as enzymes, organ tissues, glandulars, and metabolites, as well as concentrates, metabolites, constituents, extracts of dietary ingredients, oils such as krill oil and mixtures thereof;
e) homoeopathic ingredients such as those listed in the Homeopathic Pharmacopoeia of the United States Revision Service (HPRS), and mixtures thereof. It must be recognized, of course, that the HPRS is periodically updated and that the present invention includes homeopathic ingredients that may be added to the HPRS;
f) probiotics and yeast, such as bacteria selected from the group consisting of Lactobacillus (Döderlein's bacilli) such as Lactobacillus crispatus, Lactobacillus jensinii, Lactobacillus johnsonii, Lactobacillus gasseri, Enterococcus faecium, or fungi selected from the group of Saccharomycetales such as Saccharomyces boulardii.
g) hormones, such as estrogen (i.e. a natural estrogen or a synthetic compound that mimics the physiological effect of natural estrogens) including, without limitation, estradiol (17 - estradiol), estridiol acetate, estradiol benzoate, estridiol cypionate, estridiol decanoate, estradiol diacetate, estradiol heptanoate, estradiol valerate, 17a- estradiol, estriol, estriol succinate, estrone, estrone acetate, estrone sulfate, estropipate (piperazine estrone sulfate), ethynylestradiol (17a-ethynylestradiol, ethinylestradiol, ethinyl estradiol, ethynyl estradiol), ethynylestradiol 3-acetate, ethynylestradiol 3-benzoate, mestranol, quinestrol, nitrated estrogen derivatives or combinations thereof; or progestin (i.e. natural or synthetic compounds that possesses progestational activity including, without limitation, nortestosterone, ethynyltestosterone, deacetylnorgestimate, hydroxyprogesterone, 19-norprogesterone, 3P-hydroxydesogestrel, 3-ketodesogestrel (etonogestrel), acetoxypregnenolone, algestone acetophenide, allylestrenol, amgestone, anagestone acetate, chlormadinone, chlormadinone acetate, cyproterone, cyproterone acetate, demegestone, desogestrel, dienogest, dihydrogesterone, dimethisterone, drospirenone, dydrogesterone, ethisterone (pregneninolone, 17a- ethynyltestosterone), ethynodiol diacetate, fluorogestone acetate, gastrinone, gestadene, gestodene, gestonorone, gestrinone, hydroxymethylprogesterone, hydroxymethylprogesterone acetate, hydroxyprogesterone, hydroxyprogesterone acetate, hydroxyprogesterone caproate, levonorgestrel (1-norgestrol), lynestrenol (lynoestrenol), mecirogestone, medrogestone, medroxyprogesterone, medroxyprogesterone acetate, megestrol, megestrol acetate, melengestrol, melengestrol acetate, nestorone, nomegestrol, norelgestromin, norethindrone (norethisterone) (19-nor-17a-ethynyltestosterone), norethindrone acetate (norethisterone acetate), norethynodrel, norgestimate, norgestrel (d-norgestrel and dl-norgestrel), norgestrienone, normethisterone, progesterone, promegestone, quingestanol, tanaproget, tibolone, trimegestone, or combinations thereof.

One preferred class of medicaments is proton pump inhibitors. In one embodiment, the medicament is selected from the group consisting of dexlanzoprazole, esomeprazole, ilaprazole, lanzoprazole, leminoprazole, omeprazole, pantoprazole, paripiprazole, rabeprazole, tenatoprazole, and pharmaceutically acceptable combinations, salts, derivatives or enantiomers thereof.

The acid-resistant capsules may be used in any application for solid oral dosage forms in which it is advantageous to delay release of the medicament or other material in the stomach but provide release in the intestines. One such application is the delivery of medicaments that are unstable in gastric or acidic media. Another such application is to reduce gastric side effects associated with the delivery of the medicament, such as irritation, erosion, inflammation, ulcerations, pain, reflux, and other undesirable effects. Another such application is the targeted delivery of the medicament or other material to the intestines.

In one embodiment, the acid resistant hard capsules are used for delaying the release of one or more medicaments contained in said capsule when contacted with unbuffered water, preferably such that less than 20wt% of the medicament is released after 60 minutes in demineralized water.

In another embodiment, the acid resistant hard capsules are used for enteric release of one or more medicaments contained in said capsule, such that in an in vitro dissolution test, less than 10wt% of said medicament is released after 2 hours in a simulated gastric dissolution media at a pH of 1.2 and that at least 80wt% of said medicament is released after 45 minutes in a simulated intestinal dissolution media at a pH of 6.8, measured according to the USP Dissolution Apparatus 2 (paddle) method.

Once filled, the capsules can be made tamper-proof by using any conventionally used technique in the field of hard capsules to make the joint permanent. Banding or sealing are suitable techniques. Sealing is a technique well known in the field of hard shell capsules. Various alternative techniques are currently used for this purpose. A suitable procedure is disclosed for example in US 4,539,060 and US 4,656,066.

Without further elaboration, it is believed that one of ordinary skill in the art can, using the foregoing description, utilize the present invention to its fullest extent. Therefore, the following specific embodiments are to be construed as merely illustrative and not restrictive of the scope of the invention. Those of ordinary skill in the art will understand that variations of the conditions and processes of the following examples can be used.

### EXAMPLES

### Examples 1 - 8

Acid-resistant capsules were made as follows. Melt solutions were prepared by adding 153 L of water at room temperature to a 250 L vessel equipped with mixer propeller and anchor. While stirring, the HPMCAS (32kg) was dispersed in the water. Efficient dispersion and the avoidance of foaming was achieved by manual adjustment of the impeller speed as needed. Ammonia solution from a 35% w/w stock was added in small aliquots over 30 minutes to achieve an initial pH of 5.8 to 5.9. The solution was then stirred for two hours until all of the HPMCAS visually appeared to be dissolved. Additional ammonia solution was added in small aliquots over 30 minute intervals to achieve the target pH6.00 +/- 0.05. The total amount of ammonia solution added in these steps was 2.34 kg. Following stabilization of the pH, 59 L of a 21% w/w HPMC solution was then added at room temperature into the vessel. The resulting solution was then homogenized by stirring for one hour. The solution was then held at room temperature while stirring for a sufficient time to dissipate substantially all bubbles.

Capsule manufacture from the melt solution was performed by dipping pins heated to 80°C into the melt solution, which was held at 41 °C, to form a wet film on the dip molding pins through a thermogelation process. The pins were then transferred to a drying kiln held at 60°C and 20% RH. Dried capsules were then stripped from the pins, cut to an appropriate length and the cap and body were joined together.

The capsules were then placed in a controlled environmental chamber for volatile alkaline material removal. This treatment was conducted at 70°C and 60% RH for a duration of 6-18 hours. Throughout the duration of this process capsules were removed from the apparatus at selected time points to obtain samples at varied levels of residual alkaline material.

Preparation of capsules containing an additional alkaline material, mono-ethanolamine, were prepared as described above with the following exceptions. A mixed ammonia/mono-ethanolamine capsule was prepared with the formulation described above wherein the alkaline material was 1.85 kg ammonia from a 35% w/w stock and 0.158 kg of mono-ethanolamine. Removal of alkaline material was conducted at 70°C, 60% RH in a controlled environmental chamber for a duration of 8 hours. A capsule that used just mono-ethanolamine as the alkaline material was prepared as above wherein the alkaline material was 0.106 kg of mono-ethanolamine. No alkaline removal step was applied to this capsule preparation.

The degree of ionization of HPMCAS in the capsule was determined by dissolving a single capsule in 50:50 MeOH:DI H2O to a concentration of 10 mg/mL and the pH of the resulting solution was measured at 23°C using a pH electrode calibrated to pH 4.0, 7.0, and 10.0 standard buffers. The degree of ionization was calcualted as % ionization = 100/(1+ 10^{pKa-pH}) using a pKₐ value of 5.5 for HPMCAS.

The dissolution performance of the capsules was determined using a USP II dissolution apparatus. A capsule was filled with acetaminophen (APAP), mounted in a sinker and placed into the USP II dissolution apparatus filled with one liter of demineralized water held at a temperature of 37°C. The paddle speed was set at 50 rpm. Six replicate measurements were performed to provide average release of acetaminophen at selected time points.

Alternatively the measurements of APAP release were performed in a pH 1.2 buffer (0.063 M HCl) held at a temperature of 37°C for two hours. At two hours, the pH of the dissolution bath was brought to 6.8 with the addition of a disodium phosphate solution to bring the final phosphate concentration to 0.2 molar. Replicate measurements were made as described above.

The Table 1 below reports the composition of the hard shell capsules used in examples 1 to 8 in which the levels of residual ammonia in the capsules was varied using the alkaline removal step described above, or in which the composition of the alkaline material was varied using an alternative alkaline agent, mono-ethanolamine. For avoidance of doubt, amounts in wt% are by weight of a capsule shell at ambient room conditions (about 25°C and about 50%RH).

**Table 1.**

| **Example** | **1** | **2** | **3** | **4** | **5** | **6** | **7** | **8** |
|---|---|---|---|---|---|---|---|---|
| %HPMCAS¹ | 65.26 | 65.17 | 65.11 | 65.09 | 65.00 | 64.81 | 65.00 | 65.00 |
| %HPMC² | 27.97 | 27.93 | 27.90 | 27.90 | 27.86 | 27.78 | 28.00 | 28.00 |
| %NH₃ | 0.27 | 0.40 | 0.49 | 0.51 | 0.64 | 0.91 | 0 | 0.20 |
| %Mono-ethanolamine | 0 | 0 | 0 | 0 | 0 | 0 | 5.12 | 0.95 |
| %TiO₂ | 2.00 | 2.00 | 2.00 | 2.00 | 2.00 | 2.00 | 2.00 | 2.00 |
| %Moisture (water) | Balance | | | | | | | |
| Degree of Ionization (%) | 1 | 3 | 5 | 6 | 9 | 33 | - | - |
| Dissolution Performance % APAP dissolved at 1 H in pH 1.2 Buffer | 1.7 | 1.7 | 1.7 | 1.6 | 1.7 | 1.7 | 1.3 | 1.6 |
| Dissolution Performance % APAP dissolved at 2 H in pH 1.2 Buffer | 3.0 | 2.9 | 2.8 | 2.7 | 2.7 | 2.9 | 2.2 | 2.6 |
| Dissolution Performance % APAP dissolved at 1 H in pH 6.8 Buffer | - | 96.5 | - | - | - | 98.5 | 97.2 | 95.5 |
| Dissolution Performance % APAP dissolved at 2 H in pH 6.8 Buffer | - | 99.7 | - | - | - | 99.7 | 99.8 | 99.5 |
| Dissolution Performance % APAP dissolved at 1 H in demi-water | 6.0 | 7.5 | 10.3 | 10.6 | 14.6 | 84.6 | 99.6 | 12.1 |
| Dissolution Performance % APAP dissolved at 2 H in demi-water | 9.0 | 11.2 | 15.4 | 15.7 | 22.6 | 99.6 | 99.9 | 16.7 |

| | | | | | | | | |
|---|---|---|---|---|---|---|---|---|
| ¹ HPMCAS-LG, AQOAT available from Shin Etsu Chemical Co. ² HPMC 2906 Metolose available from Shin Etsu Chemical Co. | | | | | | | | |

## Claims

1. An acid resistant capsule comprising at least one hard capsule shell, said shell comprising:
at least one enteric polymer having acid groups; a film-forming aid; and an alkaline material, wherein said alkaline material is present in an amount such that said carboxylic acid groups of said enteric polymer have a degree of ionization of less than 15%.

2. The acid resistant capsule according to claim 1 wherein said degree of ionization is less than 12%, preferably less than 10%, more preferably from 0.1% to 9%.

3. The acid resistant capsule according to any of the preceding claims wherein the alkaline material is selected from the group consisting of ammonia, ethanolamine, diethanolamine, triethanolamine, lithium hydroxide, sodium hydroxide, potassium hydroxide, calcium hydroxide, magnesium hydroxide, sodium phosphate, sodium carbonate, sodium citrate, sodium ascorbate, lysine, arginine, cationic polymers, and mixtures thereof.

4. The acid resistant capsule according to any of the preceding claims wherein said alkaline material is volatile.

5. The acid resistant capsule according to any of the preceding claims wherein said alkaline material is selected from the group consisting of ammonia and ethanolamine.

6. The acid resistant capsule according to any of the preceding claims wherein said enteric polymer is selected from the group consisting of hydroxypropyl methylcellulose acetate succinate (HPMCAS), hydroxypropyl methylcellulose phthalate (HPMCP), cellulose acetate phthalate (CAP), acrylic polymers, polyvinyl acetate phthalate (PVAP), and mixtures thereof.

7. The acid resistant capsule according to any of the preceding claims wherein said film-forming aid is selected from the group consisting of hydroxypropyl methylcellulose (HPMC), methylcellulose (MC), gellan gum, carrageenan, and mixtures thereof, preferably hydroxypropyl methylcellulose (HPMC), methylcellulose (MC), and mixtures thereof, more preferably hydroxypropyl methylcellulose (HPMC).

8. The acid resistant capsule according to any of the preceding claims wherein the weight ratio of said enteric polymer to said film-forming aid is from 1.5 to 3.5, preferably from 1.8 to 2.8, more preferably from 2 to 2.5.

9. The acid resistant capsule according to any of the preceding claims wherein said alkaline material is present in said capsule in an amount of less than 7000 ppm, preferably less than 6500 ppm, more preferably from 1000 ppm to 6400 ppm, at room temperature.

10. An acid resistant capsule consisting essentially of: water present in an amount of from 1wt% to 10wt%; HPMCAS present in an amount of from 40 wt% to 90wt%; a film-forming aid selected from the group consisting of HPMC and MC present in an amount of from 10wt% to 50wt%; and an alkaline material selected from the group consisting of ammonia and ethanolamine present in an amount of less than 1wt%, more preferably less than 0.5wt%, wherein said alkaline material is present in an amount such that the carboxylic acid groups of said HPMCAS have a degree of ionization of less than 15%.

11. A dip-molding process for the manufacture of acid resistant hard capsule shells, the process comprising the steps of:
a) providing an aqueous composition comprising an enteric polymer having acid groups, a film-forming aid, and an alkaline material, said alkaline material being present in a sufficient amount so that said enteric polymer is homogeneously dispersed in said aqueous composition;
b) dipping mold pins in said aqueous composition;
c) extracting said mold pins from said aqueous composition such that a film forms over each of said mold pins;
d) drying said film to form a solid coating on each of said mold pins;
e) removing said solid coating from said mold pins to provide capsule shells; and
f) reducing the amount of said alkaline material from the capsule shells such that said acid groups of said enteric polymer in said capsule shells have a degree of ionization of less than 15%.

12. The process of claim 11 wherein said alkaline material removal step (f) is an active removal step.

13. The process according to any of claims 11 to 12 wherein the alkaline removal step (f) comprises the step of gently heating the capsule shells at a temperature of from 30°C to 80°C, preferably from 35°C to 75°C, and at a relative humidity (RH) of from 3% to 85%, preferably from 20% to 80%, more preferably from 30% to 70%.

14. The process according to any of claims 11 to 13 wherein the alkaline removal step (f) further comprises tumbling of the capsule shells, preferably simultaneously to the gentle heating.

15. The process according to any of claims 11 to 14 wherein the alkaline material removal step (f) is carried out for a predetermined period of time ranging from 1 hour to 20 hours, preferably from 1.5 hours to 18 hours, more preferably from 2 hours to 16 hours.

16. Use of an acid resistant hard capsule according to any of claims 1 to 10 for delaying the release of one or more medicaments contained in said capsule when contacted with unbuffered water, preferably such that less than 20wt% of said medicament is released after 60 minutes in demineralized water.

17. Use of an acid resistant hard capsule according to any of Claims 1 to 10 for enteric release of one or more medicaments contained in said capsule, such that less than 10wt% of said medicament is released after 2 hours at a pH of 1.2 and that at least 80wt% of said medicament is released after 45 minutes at a pH of 6.8, measured according to the USP Dissolution Apparatus 2 (paddle) method.
